# EUROPÄISCHE PATENTSCHRIFT

(11) **EP 3 372 187 B1**
(45) Veröffentlichungstag und Bekanntmachung des Hinweises auf die Patenterteilung: **31.07.2019**
(21) Anmeldenummer: 18156819.7
(22) Anmeldetag: 14.02.2018
(51) Int. Cl.: A61B 46/10, A61B 17/54

(54) **BEHANDLUNGSAUFSATZ SOWIE HANDSTÜCK MIT EINEM SOLCHEN BEHANDLUNGSAUFSATZ**
TREATMENT APPLICATION AND HANDPIECE WITH SUCH A TREATMENT APPLICATION
HAUSSE DE MANIPULATION AINSI QUE PIÈCE À MAIN POURVUE D'UNE TELLE HAUSSE DE MANIPULATION

(30) Priorität: 10.03.2017 DE 102017105190
(43) Veröffentlichungstag der Anmeldung: 12.09.2018
(73) Patentinhaber: La Fontaine, Helmut, 29604 Marbella (ES)
(72) Erfinder: La Fontaine, Helmut, 29604 Marbella (ES)
(74) Vertreter: Geitz Truckenmüller Lucht Christ

(56) Entgegenhaltungen:
- WO-A1-2016/029332
- DE-A1-102008 031 132
- US-A1- 2010 268 249

## Beschreibung

Die vorliegende Erfindung betrifft einen Behandlungsaufsatz zur Verbindung mit dem Handstück eines medizinischen oder kosmetischen Hautbehandlungsgeräts, umfassend einen Gehäuseaufsatz sowie ein in dem Gehäuseaufsatz beweglich aufgenommenes Behandlungselement mit einem Behandlungskopf und einer Schubstange zur lösbaren Verbindung mit einem Antrieb des Handstücks, sowie ein Handstück mit einem solchen Behandlungsaufsatz.

Solche Behandlungsaufsätze sind bereits im Stand der Technik bekannt. So greift etwa die EP 2082696A1 auf einen solchen Behandlungsaufsatz in Form einer Abrasionseinheit zurück, die von einer Schutzhülse umgeben ist. Die Schutzhülse und die Abrasionseinheit werden gemeinsam als sterile EinwegTeile geliefert und bei jeder Behandlung ausgetauscht. Hierdurch ist sichergestellt, dass alle Teile einer solchen Abrasionsvorrichtung mit Hautkontakt unbedenklich sind.

Bei der Microdermabrasion werden die obersten Hautschichten durch eine rotatorische Schleifbewegung eines Abrasionsmittels abgetragen und hierdurch die Haut zur Erneuerung angeregt.

Auch in der DE 10 2004 032 892 A1 desselben Anmelders wird auf die Verwendung eines Behandlungsaufsatzes abgestellt, wobei im Vergleich zu dem vorgenannten Stand der Technik das Behandlungselement axiale Stoßbewegungen ausführt und nicht drehbar gelagert ist. Dies geschieht im Rahmen einer Perforation, bei der mithilfe sehr dünner Nadeln feine Löcher in die Haut gestoßen werden, so dass die Haut durchlässig für anschließend aufzutragende Wirkstoffe ist.

In beiden Fällen kann es passieren, dass Wirkstoffe von den einzelnen Behandlungen oder auch Hautpartikel oder Körperflüssigkeiten an dem Handstück anhaften, so dass auch dieses mit großem Aufwand gereinigt werden muss. Die Verwendung von Einweg-Behandlungsmitteln entbindet also nicht von aufwändigen Reinigungsmaßnahmen.

Um dies zu umgehen, wäre es erforderlich, das ganze Handstück in einer Schutzhülle einzupacken, die ein Verunreinigen des Handstücks verhindern würde. So ist bereits aus der CN 102038479 A bekannt, ein Endoskop mit einer Einweg-Schutzhülle zu verwenden, die nach jeder Behandlung oder Untersuchung abgestreift und entsorgt wird. Aufgrund der vielen Einwegteile und der komplexen Struktur sieht die genannte Schrift lediglich eine Art Überwurf vor, der über das Endoskop gebreitet wird und unter dem der ausführende Mediziner praktisch in herkömmlicher Weise arbeitet.

Die JP H 03289950 A betrifft weiter eine Ultraschallsonde, welche mit einer Schutzhülle überzogen wird, die neben der Verschmutzung nach innen auch das Austreten von Ultraschallflüssigkeit und Glassplittern verhindern soll, falls ein Ultraschallfenster im Betrieb zerbrechen sollte. Hierbei handelt es sich um eine dicht anliegende Hülle in der Art eines Präservativs.

Die WO 2016/029332 beschreibt ein elektrisches Gerät zur Microdermabrasion, das aus einem Handstück mit Motor, einem Arbeitskopf, und einer Plastikschutzkappe für den Arbeitskopf besteht. Zudem verfügt das Gerät über einen Antrieb mit mehreren Gängen.

Die DE 10 2008 031 132 beschreibt einen elektrischen Trockenrasierer, der aus einem Handstück mit Antriebsmotor und Arbeitskopf besteht. Dabei ist der Kopf seitlich schwenkbar ausgebildet.

Aus der US 9,017,851 B2 ist es ferner bekannt, ein Netzgerät mit einer Anschlusselektrode zu versehen und mit einer Schutzhülle zu umgeben. Das Sterile Operationsgerät wird dann mit Strom versorgt, indem es auf die Elektrode aufgesetzt wird, die wiederum so geformt ist, dass sie die Schutzhülle hierbei durchstößt.

Schließlich ist aus der WO 94/16763 A1 und ähnlich aus der WO 98/56452 A1 bekannt, Schlauchkupplungen und -abzweigungen in sterile Schutzumschläge einzupacken, so dass diese sicher vor Verunreinigungen sind.

Die genannten Schutzhüllen lassen sich jedoch nicht auf die eingangs genannten Hautbehandlungsgeräte anwenden. Zwar ist es möglich, diesen Präservative überzuziehen, aber dann kommen die Behandlungsflächen zur Perforation und Abrasion nicht mehr in Eingriff mit der Haut. Ein reiner Überwurf hingegen scheint keine geeignete Lösung für das Problem zu sein, da die Spitze des Handstücks selbst die Behandlung durchführt. Die Behandlung würde dadurch mehr oder minder direkt unter dem Überwurf stattfinden, so dass sich keine Verbesserung einstellen würde. Die zuletzt genannten sterilen Schutzumschläge hingegen sind nur mit einem großen Aufwand um ein Handstück herum anzubringen und wieder zu entfernen.

Vor diesem Hintergrund liegt der vorliegenden Erfindung die Aufgabe zu Grunde, einen sterilen Schutz für ein Handstück eines medizinischen oder kosmetischen Hautbehandlungsgeräts zu schaffen, welcher leicht appliziert und entfernt werden kann und bei dem der Benutzer zu einem regelmäßigen Erneuern angehalten ist.

Gelöst wird dies durch einen Behandlungsaufsatz gemäß den Merkmalen des Anspruchs 1, sowie durch ein Handstück für ein medizinisches oder kosmetisches Hautbehandlungsgerät gemäß den Merkmalen des nebengeordneten Anspruchs 9. Weitere sinnvolle Ausgestaltungen eines solchen Behandlungsaufsatzes oder eines entsprechenden Handstücks können den jeweiligen abhängigen Ansprüchen entnommen werden.

Erfindungsgemäß ist es vorgesehen, einen Behandlungsaufsatz mit einem aufgerollten Materialschlauch bereitzustellen, der bei jeder Behandlung getauscht wird. Dieser wird nach der Verbindung des Behandlungsaufsatzes mit dem Handstück entlang des Gehäuses des Handstücks abgeroll, so dass der den Spalt zwischen dem Behandlungsaufsatz und dem Handstück überdeckt und abdichtet und ein Eindringen von Lymphe oder Blut in das Innere des Handstücks verhindert wird. Hierdurch ergibt es sich ferner, dass mit dem Tausch des Behandlungsaufsatzes auch ein Tausch des sterilen Materialschlauchs erfolgt, so dass die nach außen zugängliche Oberfläche des Handstücks bei jeder Behandlung auch steril ist. Eine umständliche Sterilisierung des Handstücks kann dadurch entfallen.

Der Behandlungsaufsatz besteht, wie auch bereits beim Stand der Technik, aus einem Gehäuseaufsatz und einem Behandlungselement. Das Behandlungselement kann beispielsweise eine Nadelplatte oder eine Schleiftablette oder ähnliches sein, mit dem eine Hautbehandlung durchgeführt wird. Die Erfindung erlaubt es hierbei explizit, sowohl rotatorische Bewegungen des Behandlungselements, als auch translatorische Bewegungen auszuführen, so dass praktisch alle Arten von Hautbehandlungsmitteln eingesetzt werden können. Der Gehäuseaufsatz stellt eine Erweiterung des Handstücks dar, so dass die beiden Elemente leicht miteinander verbunden und ein auf dem Gehäuseaufsatz aufgewickelter Materialschlauch über das Ende des Gehäuseaufsatzes hinweg über das Handstück abgerollt werden kann. Die Schutzhülle, welche in dem Materialschlauch ausläuft, ist elastisch und legt sich aufgrund ihrer Elastizität eng an das Handstück an. Hierdurch stört die Schutzhülle den Benutzer nicht, sondern funktioniert wie ein Schutzfilm über dem Handstück. Im Bereich der Spitze des Gehäuseaufsatzes besitzt die Schutzhülle eine Durchlassöffnung mit einem Rand, der sich dichtend um eine Schubstange des Behandlungselements herumlegt. Eine besondere Ausgestaltung des Rands kann, muss aber nicht vorgesehen sein. In der einfachsten Ausgestaltung ist die Schutzhülle nur eingeschnitten. Ein Behandlungskopf des Behandlungselements verbleibt also außerhalb der Schutzhülle und bleibt so in der Lage, mit der zu behandelnden Haut in Eingriff zu treten. Lediglich mit seiner Schubstange, welcher für eine Wirkverbindung des Behandlungskopfes mit einem Antrieb des Handstücks sorgt, durchgreift das Behandlungselement somit die Schutzhülle, ist aber sicher abgedichtet gegenüber der Behandlungsfläche.

Nimmt die behandelnde Person das Handstück also in Betrieb, so wird sie zunächst einen Behandlungsaufsatz aus einer sterilen Verpackung, vorzugsweise aus einer Blisterverpackung, entnehmen und auf das Handstück aufsetzen. Hierbei ist darauf zu achten, dass die Schubstange in Wirkverbindung mit dem Antrieb gerät und dass der Gehäuseaufsatz auf dem Gehäuse aufgesetzt wird. Dann wird die Schutzhülle, die aufgerollt auf der Außenseite des Gehäuseaufsatzes vorgehalten wird, weg von dem Behandlungskopf über das Gehäuse des Handstücks abgerollt und bildet dadurch einen Schutzfilm über dem Gehäuse des Handstücks. Durch diesen Schutzfilm hindurch kann das Handstück in gewohnter Weise bedient werden.

Dabei kann es zudem sinnvoll sein, wenn das Gehäuse des Handstücks mit dem Gehäuseaufsatz einen fließenden Übergang bildet, so dass sich über diesen Übergang hinweg der Materialschlauch der Schutzhülle leicht abrollen lässt.

Hinter dem Behandlungskopf bildet sich hierbei eine Kavität aus, in die das Behandlungselement eingesetzt ist. In dieser Kavität schließt sich die Schutzhülle um die Schubstange des Behandlungselements und dichtet dieses auch von dieser Seite her ab. Aufgrund der Elastizität der Schutzhülle kann das Behandlungselement nun Hubbewegungen ohne Weiteres ausführen, da die Längenänderungen von der elastischen Schutzhülle mitgemacht werden. Auch oszillierende Kreisbewegungen hin und her können so ohne Weiteres ausgeführt werden, wohingegen für Rotationsbewegungen der Rand um den Hals herum genügend gleitend sein muss. Entweder leistet dies der Rand aufgrund der Materialwahl ohnehin, oder der Schubstange wird eine dichtende Manschette umgelegt, die gegenüber der Schubstange dichtend wirkt, aber gegenüber dem Rand der Durchlassöffnung der Schutzhülle drehfest ist.

Nach der Behandlung wird die Schutzhülle wieder aufgerollt und zusammen mit dem Gehäuseaufsatz und dem Behandlungselement entsorgt.

In sinnvoller Weiterbildung der Erfindung kann der Gehäuseaufsatz an seinem vom Behandlungskopf weg weisenden Ende Rastmittel aufweisen, mit denen er mit dem Gehäuse des Handstücks verbunden werden kann. Hierbei kann es sich etwa um eine klemmende Verbindung handeln, wenn der Gehäuseaufsatz so konstruiert ist, dass er aufgrund von Reibschluss mit dem Handstück zusammenwirkt. Jedoch sind auch Schraubgewinde als Verschlussmittel denkbar, sowie ein Bajonettverschluss. Soweit sich andere bekannte Verschlussmechanismen hierauf ohne Weiteres anwenden lassen, sind diese ebenfalls ausdrücklich von der Erfindung mit umfasst.

Um den Materialschlauch in seinem aufgerollten Zustand besser an dem Gehäuseaufsatz zu fixieren und seine Ausgangsposition zu definieren, kann der Gehäuseaufsatz ferner eine umlaufende Sicke aufweisen, in welcher der aufgerollte Materialschlauch aufgenommen ist. Hierbei befindet sich der Materialschlauch nicht vollständig in der Sicke, sondern wird durch diese lediglich grob gegen ein Abrutschen fixiert.

Das Behandlungselement kann zudem mit einigem Vorteil dem Behandlungsaufsatz, mithin dem Gehäuseaufsatz, unverlierbar zugeordnet sein. Insbesondere kann dies durch ein Element erfolgen, das am freien Ende der Schubstange angeordnet ist, um eine axiale Kraftübertragung zu ermöglichen. Ein solches Element kann etwa ein Rundkopf sein, der in eine korrespondierende Kugelpfanne einer Schubstange des Antriebs eingreifen kann. Hierzu muss entweder die Kugelpfanne oder der Rundkopf eine Federkerbe aufweisen, so dass die benötigte Elastizität für eine hier realisierte Clipverbindung vorhanden ist.

Die Schubstange des Antriebs kann ferner aus zwei aufeinander folgenden Teilen bestehen, die unter Zwischenlage einer Feder zu der Schubstange zusammengefügt sind. Bei einem zu starken Druck der Behandlungsvorrichtung auf die Haut wird die Druckfeder gestaucht und verhindert, dass die auf das Handstück ausgeübte Kraft direkt auf die Haut übertragen wird. Wird die Kraft auf das Handstück größer als eine durch die Federkonstante der Druckfeder vorgegebene Kraft, so wird dem Ausrückvorgang der Schubstange des Antriebs eine Stauchung der Feder überlagert, so dass der Schub des Antriebs nicht vollständig auf die Haut übertragen wird.

Schließlich kann einem solchen Element zusätzlich oder ersatzweise ein Element zur Drehmitnahme zugeordnet sein, so dass bei Handstücken, deren Behandlungsaufsatz für eine Drehbewegung hergerichtet sein muss, die Schubstange des Antriebs auch diese Drehbewegung übertragen kann.

Die vorstehend beschriebene Erfindung wird im Folgenden anhand eines Ausführungsbeispiels näher erläutert.

Es zeigen
- Figur 1: einen Behandlungsaufsatz bestehend aus einem Gehäuseaufsatz, einem Behandlungselement und einer dazwischen liegenden Schutzhülle, in einer seitlichen Querschnittsdarstellung,
- Figur 2: den Behandlungsaufsatz gemäß Figur 1, welcher auf einem passenden Handstück aufgesetzt und mit diesem Wirkverbunden ist, in einer seitlichen Querschnittsdarstellung,
- Figur 3: eine Variante des Behandlungsaufsatzes gemäß Figur 2 in einer seitlichen Querschnittsdarstellung,
- Figur 4: das Handstück gemäß Figur 2 mit aufgesetztem Behandlungsaufsatz, bei dem ein Teil des Materialschlauchs der Schutzhülle über das Gehäuse des Handstücks abgerollt ist, in einer seitlichen Querschnittsdarstellung, sowie
- Figur 5: das Handstück gemäß Figur 2 mit aufgesetztem Behandlungsaufsatz, bei dem der Materialschlauch der Schutzhülle vollständig über das Gehäuse des Handstücks abgerollt ist, in einer seitlichen Querschnittsdarstellung.

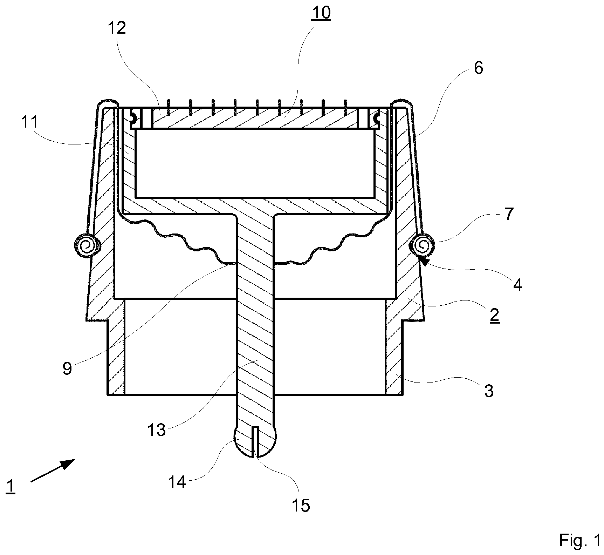

Figur 1 zeigt einen Behandlungsaufsatz 1, bestehend aus einem Gehäuseaufsatz 2 und einem Behandlungselement 10. Das Behandlungselement 10 umfasst einen Behandlungskopf 11 mit einer mit Nadeln versehenen Behandlungsfläche 12. An dem gegenüberliegenden Ende des Behandlungskopfes 11 schließt sich eine Schubstange 13 an, welche an ihrem freien Ende einen Rundkopf 14 mit einer Federkerbe 15 aufweist, welcher wiederum mit einem Antrieb eines mit dem Behandlungsaufsatz zu verbindenden Handstück wirkverbunden werden kann.

Zwischen dem Gehäuseaufsatz 2 und dem Behandlungskopf 11 ist eine elastische Schutzhülle 6 angeordnet, welche über eine Durchlassöffnung von der Schubstange 13 des Behandlungselements 10 durchdrungen wird. Die Durchlassöffnung weist hierbei einen Rand 9 auf, welcher wie die restliche Schutzhülle 6 elastisch ist und sich um die Schubstange 13 herum dichtend an diese anlegt. Die Schutzhülle läuft um den Behandlungskopf 11 herum aus einer diesen aufnehmenden Kavität des Gehäuseaufsatzes 2 heraus, umrundet eine Vorderkante des Gehäuseaufsatzes 2 und legt sich an dessen Außenwand an. Dort ist ein von der Schutzhülle 6 gebildeter aufgerollter Materialschlauch 7 platziert, dessen Position von einer Sicke 4 des Gehäuseaufsatzes 2 vorgegeben wird.

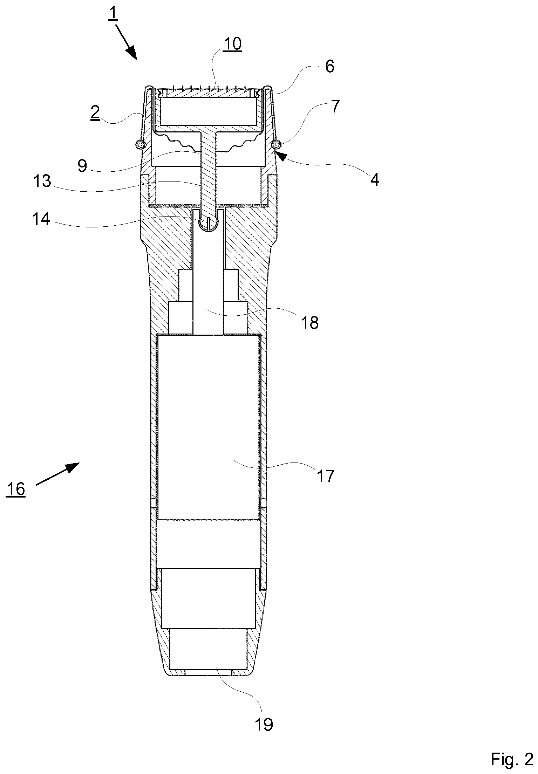

Figur 2 zeigt nun einen ersten Schritt der Vorbereitung eines Handstücks 16, auf das der Behandlungsaufsatz gemäß Figur 1 aufgesetzt wird. Zunächst wird der Gehäuseaufsatz 2 mit seinem von dem Behandlungskopf 11 entfernt liegenden Ende auf eine korrespondierende Aufnahme des Gehäuses des Handstücks 16 aufgesteckt, wo der Gehäuseaufsatz 2 durch Reibschluss auf dem Gehäuse des Handstücks 16 gehalten wird. Hierbei wird gleichzeitig die Schubstange 13 des Behandlungselements 10 mit dem Rundkopf 14 in eine Kugelpfanne einer Schubstange 18 Handstücks 16 hineingedrückt, so dass eine lösbare Clipverbindung entsteht. Das Handstück 16 weist ferner einen Antrieb 17 auf, welcher die Schubstange 18 in axiale Hubbewegungen versetzen kann. Die Stromversorgung des elektrischen Antriebs 17 erfolgt über eine hier nicht dargestellte Stromquelle, die über Anschlussmittel 19 mit dem Handstück 16 verbunden werden kann.

Erfolgt dies und wird das Handstück 16 dann in Betrieb genommen, so führt der Antrieb 17 Hubbewegungen aus und das Behandlungselement 10 wird in Hubbewegungen abwechselnd in die Kavität des Gehäuseaufsatzes 2 hineingezogen und wieder heraus, gegen die Haut einer zu behandelnden Person gedrückt. Der Rand 9 der Schutzhülle 6 ist hierbei flüssigkeitsdicht, aufgrund der Elastizität der Schutzhülle 6 macht diese die Hubbewegungen mit und behindert dadurch den Betrieb des Handstücks 16 nicht. Die Oberfläche der Schutzhülle ist ferner entweder so weit von den Flanken des Behandlungskopfes 11 entfernt, dass dessen reibungslose und damit bremsfreie Bewegung ermöglicht ist, oder weist eine Beschichtung oder Bestäubung auf, welche diese verhindert.

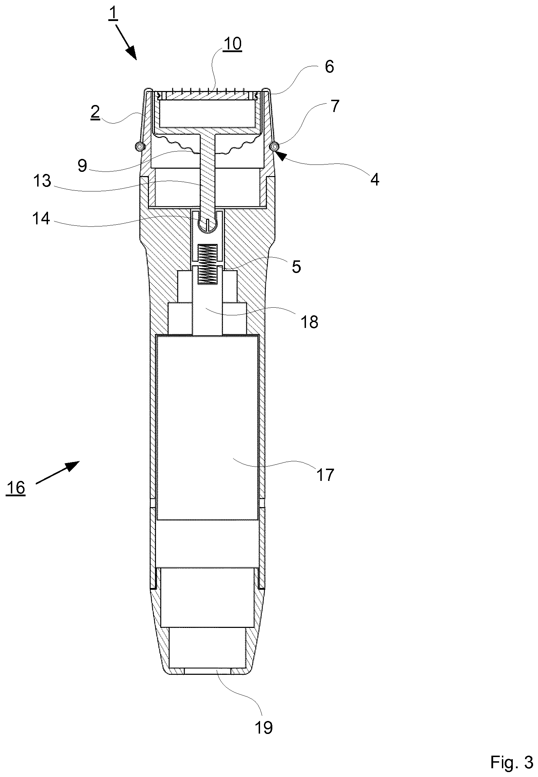

Figur 3 zeigt eine Variante des Handstücks 16 mit einem Behandlungsaufsatz, wobei der Antrieb 17 eine Schubstange 18 mit einer eingesetzten Druckfeder 5 aufweist. Der Antrieb 17 schiebt die Schubstange 18 auf die Haut einer zu behandelnden Person zu, so dass die Nadeln des Behandlungselements in die Haut der zu behandelnden Person eindringen können. Um einem zu starken Druck einer behandelnden Person auf das Handstück und damit auf die Haut der zu behandelnden Person zu vermeiden, ist die Druckfeder 5 so eingestellt, dass sie bei einem Maximaldruck beginnt, sich zu stauchen. Aufgrund dieser Stauchung wird der Druck auf die Haut reduziert, so dass nicht der gesamte ausgeübte Druck der behandelnden Person auf die Haut der zu behandelnden Person übertragen wird.

Grundsätzlich wäre das Handstück 16 nun betriebsbereit, jedoch würde nun die Gefahr bestehen, dass ein verunreinigtes Handstück 16 in Kontakt mit der Haut einer zu behandelnden Person in Kontakt kommt, so dass in den folgenden beiden Figuren gezeigt wird, wie erfindungsgemäß eine sterile Schutzhülle über dem Handstück 16 angebracht werden kann. Ebenfalls vermeidet eine solche Schutzhülle, dass Blut oder Lymphe von der Behandlung in den Materialspalt zwischen Handstück 16 und Behandlungsaufsatz eintritt und das Handstück 16 stark verunreinigt.

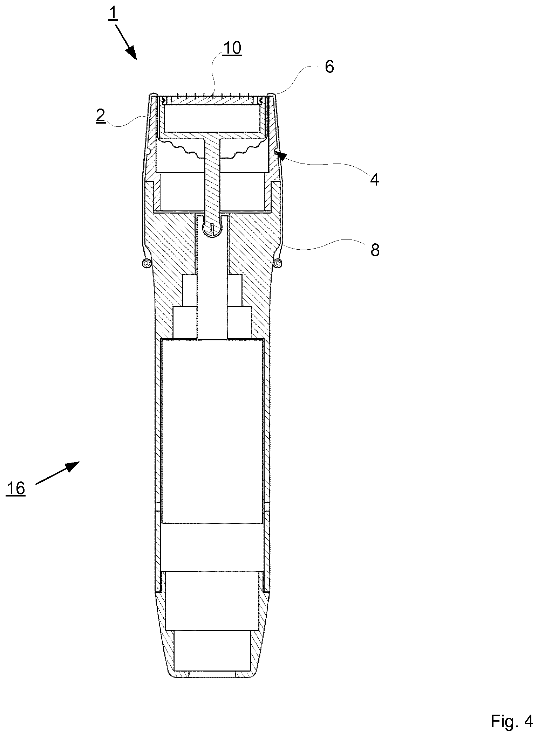

Hierzu wird, wie in Figur 4 gezeigt, der ursprünglich in der Sicke 4 positionierte Materialschlauch 8 abgerollt und hierbei mit den Fingern über das Handstück gestreift. Aufgrund eines fließenden, flächenbündigen Übergangs zwischen Gehäuseaufsatz 2 und Handstück 16 kann der Materialschlauch 8 über diesen Übergang hinweggestreift werden, so dass dieser empfindliche Übergangsbereich zusätzlich abgedichtet ist. Es wäre bereits ausreichend, den Materialschlauch 8 bis über diesen Übergang zu ziehen, um die Dichtigkeit der Anordnung zu gewährleisten. Je nach der realisierten Länge des Materialschlauchs 8 kann dieser auch weiter abgerollt werden, wie im Folgenden gezeigt.

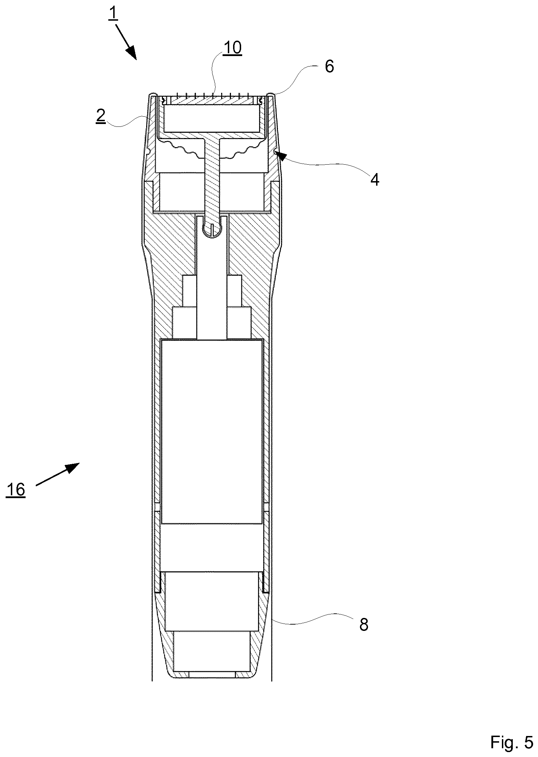

Wie in Figur 5 gezeigt, wird der Materialschlauch 8 vollständig über das Handstück abgerollt und legt sich aufgrund seiner Elastizität an dieses an. Während der Behandlung ist jedes Teil des Handstücks 16 und des Gehäuseaufsatzes 2 hinter der Schutzhülle 6 eingeschlossen, mit Ausnahme des weit von dem Behandlungskopf 11 entfernten elektrischen Anschlusses und des Behandlungskopfes 11 selbst. Letzterer muss jedoch auch in Eingriff mit der Haut gebracht werden und wird hierfür steril geliefert.

Vorstehend beschrieben ist somit ein Behandlungsaufsatz und ein Handstück eines medizinischen oder kosmetischen Hautbehandlungsgeräts zur Verwendung desselben, bei dem ein steriler Schutz für das Handstück geschaffen wird, welcher leicht appliziert und entfernt werden kann und bei dem der Benutzer zu einem regelmäßigen Erneuern angehalten ist.

### BEZUGSZEICHENLISTE

- 1: Behandlungsaufsatz
- 2: Gehäuseaufsatz
- 3: Rastmittel
- 4: Sicke
- 5: Druckfeder
- 6: Schutzhülle
- 7: aufgerollter Materialschlauch
- 8: abgerollter Materialschlauch
- 9: Rand
- 10: Behandlungselement
- 11: Behandlungskopf
- 12: Behandlungsfläche
- 13: Schubstange
- 14: Rundkopf
- 15: Federkerbe
- 16: Handstück
- 17: Antrieb
- 18: Schubstange
- 19: Anschlussmittel

## Patentansprüche

1. Behandlungsaufsatz (1) zur Verbindung mit dem Handstück (16) eines medizinischen oder kosmetischen Hautbehandlungsgeräts, umfassend einen Gehäuseaufsatz (2) sowie ein in dem Gehäuseaufsatz (2) beweglich aufgenommenes Behandlungselement (10) mit einem Behandlungskopf (11) und einer Schubstange (13) zur lösbaren Verbindung mit einem Antrieb (17) des Handstücks (16),
**dadurch gekennzeichnet, dass** der Behandlungskopf (11) unter Zwischenlage einer elastischen Schutzhülle (6) in einer Kavität des Gehäuseaufsatzes (2) aufgenommen ist, welche einen aufgerollt an der Außenwand des Gehäuseaufsatzes (2) anliegenden Materialschlauch (7, 8) ausbildet und eine Durchlassöffnung aufweist, deren Rand (9) an der Schubstange (13) des Behandlungselements (10) dichtend anliegt.

2. Behandlungsaufsatz gemäß Anspruch 1, **dadurch gekennzeichnet, dass** der Gehäuseaufsatz (2) an seinem von dem Behandlungskopf (11) weg weisenden Ende Rastmittel aufweist, welche mit Gegenrastmitteln des Handstücks (16) zusammenwirken können.

3. Behandlungsaufsatz gemäß Anspruch 2, **dadurch gekennzeichnet, dass** es sich bei den Rastmitteln und Gegenrastmitteln um Elemente eines Klemmverschlusses, Schraubverschlusses oder Bajonettverschlusses handelt.

4. Behandlungsaufsatz gemäß einem der vorhergehenden Ansprüche, **dadurch gekennzeichnet, dass** der Außenwand des Gehäuseaufsatzes (2) eine umlaufende Sicke (4) zur Aufnahme des aufgerollten Materialschlauchs (7) zugeordnet ist.

5. Behandlungsaufsatz gemäß einem der vorhergehenden Ansprüche, **dadurch gekennzeichnet, dass** das Behandlungselement (10) in dem Gehäuseaufsatz (2) unverlierbar aufgenommen ist.

6. Behandlungsaufsatz gemäß einem der vorhergehenden Ansprüche, **dadurch gekennzeichnet, dass** die Schubstange (13) des Behandlungselements (10) an ihrem freien Ende ein Element zur axialen Kraftübertragung aufweist.

7. Behandlungsaufsatz gemäß Anspruch 5, **dadurch gekennzeichnet, dass** es sich bei dem Element zur axialen Kraftübertragung um einen Rundkopf (14) handelt, welcher vorzugsweise eine Federkerbe (15) aufweist.

8. Behandlungsaufsatz gemäß einem der vorhergehenden Ansprüche, **dadurch gekennzeichnet, dass** die Schubstange (13) an ihrem freien Ende wenigstens ein Element zur Drehmitnahme aufweist.

9. Handstück (16) für ein medizinisches oder kosmetisches Hautbehandlungsgerät, das Handstück (16) umfassend einen Behandlungsaufsatz (1) gemäß einem der vorhergehenden Ansprüche sowie ein Gehäuse (20) mit einem in dem Gehäuse (20) aufgenommenen elektrischen Antrieb (17), welcher über eine Schubstange (18) mit der Schubstange (13) des Behandlungselements (10) wirkverbunden ist.

10. Handstück gemäß Anspruch 9, **dadurch gekennzeichnet, dass** die Schubstange 18 des Antriebs (17) in zwei in Wirkrichtung unter Zwischenlage einer Druckfeder (5) aufeinander folgende Teilstangen unterteilt ist.

11. Handstück gemäß einem der Ansprüche 9 oder 10, **dadurch gekennzeichnet, dass** die Außenkontur des Gehäuseaufsatzes (2) mit einer Außenkontur des Gehäuses (20) einen fließenden Übergang bildet, wobei der Materialschlauch (8) über diesen fließenden Übergang hinweg entlang des Gehäuses (20) abgerollt ist.

## Claims

1. A treatment attachment (1) for connection to the handpiece (16) of a medical or cosmetic skin treatment device, comprising a housing attachment (2) and a treatment element (10), movably accommodated in the housing attachment (2), with a treatment head (11) and a push rod (13) for detachable connection to a drive (17) of the handpiece (16), **characterized in that** the treatment head (11) is accommodated in a cavity in the housing attachment (2) with an elastic protective casing (6) situated in between, the protective casing forming a material hose (7, 8) which when rolled out lies against the outer wall of the housing attachment (2), and having an outlet opening whose edge (9) rests against the push rod (13) of the treatment element (10) in a sealing manner.

2. The treatment attachment according to Claim 1, **characterized in that** the housing attachment (2) on its end pointing away from the treatment head (11) has locking means that may interact with counterlocking means of the handpiece (16).

3. The treatment attachment according to Claim 2, **characterized in that** the locking means and counterlocking means are elements of a clamp lock, screw lock, or bayonet lock.

4. The treatment attachment according to one of the preceding claims, **characterized in that** a circumferential rim (4) for accommodating the rolled-out material hose (7) is associated with the outer wall of the housing attachment (2).

5. The treatment attachment according to one of the preceding claims, **characterized in that** the treatment element (10) is captively held in the housing attachment (2).

6. The treatment attachment according to one of the preceding claims, **characterized in that** the push rod (13) of the treatment element (10) at its free end has an element for axial transmission of force.

7. The treatment attachment according to Claim 5, **characterized in that** the element for axial transmission of force is a round head (14) preferably having a spring groove (15).

8. The treatment attachment according to one of the preceding claims, **characterized in that** the push rod (13) at its free end has at least one element for rotational entrainment.

9. A handpiece (16) for a medical or cosmetic skin treatment device, the handpiece (16) comprising a treatment attachment (1) according to one of the preceding claims, and a housing (20) with an electric drive (17) that is accommodated in the housing (20) and is operatively connected to the push rod (13) of the treatment element (10) via a push rod (18).

10. The handpiece according to Claim 9, **characterized in that** the push rod (18) of the drive (17) is divided into partial rods that follow one another in succession in the direction of action, with a compression spring (5) situated in between.

11. The handpiece according to one of Claims 9 or 10, **characterized in that** the outer contour of the housing attachment (2) forms a smooth transition with an outer contour of the housing (20), wherein the material hose (8) is unrolled along the housing (20) via this smooth transition.

## Revendications

1. Embout de traitement (1) à raccorder à la pièce à main (16) d'un appareil de traitement cutané médical ou cosmétique, comprenant un embout formant boîtier (2) ainsi qu'un élément de traitement (10), logé de manière mobile dans l'embout formant boîtier (2), avec une tête de traitement (11) et une tige de poussée (13) à raccorder de manière amovible à un entraînement (17) de la pièce à main (16),
**caractérisé en ce que** la tête de traitement (11) est logée dans une cavité de l'embout formant boîtier (2) par interposition d'une gaine de protection élastique (6) qui constitue un flexible (7, 8) appliqué contre la paroi extérieure en étant enroulé et présente une ouverture de passage dont le bord (9) s'applique de manière étanche à la tige de poussée (13) de l'élément de traitement (10).

2. Embout de traitement selon la revendication 1, **caractérisé en ce que** l'embout formant boîtier (2) présente à son extrémité détournée de la tête de traitement (11) des moyens de verrouillage pouvant coopérer avec des moyens de verrouillage complémentaires de la pièce à main (16).

3. Embout de traitement selon la revendication 2, **caractérisé en ce que** les moyens de verrouillage et les moyens de verrouillage complémentaires sont des éléments d'une fermeture par serrage, d'une fermeture par vissage ou d'une fermeture à baïonnette.

4. Embout de traitement selon l'une quelconque des revendications précédentes, **caractérisé en ce qu'**un collet périphérique (4) destiné à recevoir le flexible enroulé (7) est attribué à la paroi extérieure de l'embout formant boîtier (2).

5. Embout de traitement selon l'une quelconque des revendications précédentes, **caractérisé en ce que** l'élément de traitement (10) est reçu de manière imperdable dans l'embout de boîtier (2).

6. Embout de traitement selon l'une quelconque des revendications précédentes, **caractérisé en ce que** la tige de poussée (13) de l'élément de traitement (10) présente à son extrémité libre un élément pour la transmission de force axiale.

7. Embout de traitement selon la revendication 5, caractérisé en ce l'élément pour la transmission de force axiale est une tête ronde (14) présentant de préférence une encoche pour ressort (15).

8. Embout de traitement selon l'une quelconque des revendications précédentes, **caractérisé en ce que** la tige de poussée (13) présente à son extrémité libre au moins un élément pour un entraînement en rotation.

9. Pièce à main (16) pour un appareil de traitement cutané médical ou cosmétique, ladite pièce à main (16) comprenant un embout de traitement (1) selon l'une quelconque des revendications précédentes ainsi qu'un boîtier (20) avec un entraînement électrique (17) logé dans ledit boîtier (20) et en liaison fonctionnelle avec la tige de poussée (13) de l'élément de traitement (10) par l'intermédiaire de la tige de poussée (18) .

10. Pièce à main selon la revendication 9, **caractérisée en ce que** la tige de poussée (18) de l'entraînement (17) est divisée en deux tiges partielles successives dans la direction d'action avec interposition d'un ressort de pression (5).

11. Pièce à main selon l'une quelconque des revendications 9 ou 10, **caractérisée en ce que** le contour extérieur de l'embout formant boîtier (2) constitue avec un contour extérieur du boîtier (20) une transition progressive, le flexible (8) étant déroulé le long du boîtier (20) en passant par ladite transition progressive.
